# EUROPEAN PATENT APPLICATION

(11) **EP 4 458 320 A1**
(43) Date of publication of application: **06.11.2024**
(21) Application number: 22914639.4
(22) Date of filing: 26.12.2022
(51) Int. Cl.: A61F 2/07

(54) **BRANCH STENT**

(30) Priority: 30.12.2021 CN 202111664817
(71) Applicant: Lifetech Scientific (Shenzhen) Co., Ltd., Shenzhen, Guangdong 518063 (CN)
(72) Inventor: LIU, Tangjie, Shenzhen, Guangdong 518063 (CN); XIAO, Benhao, Shenzhen, Guangdong 518063 (CN)
(74) Representative: Michalski Hüttermann & Partner Patentanwälte mbB
(86) International application number: PCT/CN2022/141842
(87) International publication number: WO 2023/125385

(57) **Abstract**

A branch stent, including a stent body and a skirt structure disposed on the stent body. The stent body includes a main body stent and a main body covering film disposed on the main body stent. The skirt structure includes a sealing stent segment connected to the stent body and a fixing stent segment connected to the sealing stent segment, and the skirt structure is provided with a developing structure. For the branch stent, a branch opening of a vascular stent is sealed by the sealing stent segment to prevent endoleaks. The fixing stent segment can be folded, and is automatically folded after being released from a delivery sheath, to prevent the branch stent from moving. Also, the skirt structure is provided with the developing structure for displaying the state of the skirt structure after being released from the delivery sheath, making it convenient for an operator to observe.

## Description

### Technical Field

The embodiments relate to medical instruments, and to a branch stent.

### Background

Aortic aneurysm and aortic dissection are currently serious diseases that endanger human life. If there is no active treatment, the aortic aneurysm and the aortic dissection will continue to increase, and finally rupture, resulting in serious complications and death. With the increasing number of patients with hypertension, hyperlipidemia and diabetes (hyperglycaemia), incidences of the aortic aneurysm and the aortic dissection are significantly increased.

A traditional open surgery treating the aortic aneurysm and the aortic dissection features a heavy wound, a high mortality, a long surgery time, a high incidence of post-surgery complications and high difficulty of the surgery. While endovascular treatment features a minimal injury, less post-surgery complications, a short surgery time, low difficulty of the surgery, etc., the endovascular treatment has gradually become a current main method for treating the aortic aneurysm and the aortic dissection. By implanting a covered stent in an aorta, a vascular lesion is isolated outside the covered stent, and a blood flow is restricted from flowing through an interior of the covered stent, so as to achieve a purpose of protecting a blood vessel.

When the covered stent is applied, a main body stent and a branch stent are often used in combination, and the presence of endoleaks is one of common problems during use in combination. A skirt stent is a branch stent specially applied to reconstruction of a branch channel, and the skirt has a good endoleaks prevention effect if it is used with the main body stent in combination by special endoleaks prevention design. An expansion state of the skirt in the surgery directly determines the endoleaks prevention effect of the branch stent.

Therefore, there is a need to solve the problem that the expansion state of the skirt of the branch stent is difficult to be observed after the branch stent is implanted.

### Summary

An objective of the present embodiments is to solve at least the problem that the state of the skirt is difficult to be observed after a branch stent with a skirt is implanted. Embodiments provide a branch stent including a stent body and a skirt structure disposed on the stent body; the stent body includes a main body stent and a main body covering film disposed on the main body stent; and the skirt structure includes a sealing stent segment connected to the stent body and a fixing stent segment connected to the sealing stent segment, and the skirt structure is configured to provide with a developing structure.

The branch stent of embodiments may be placed at a branch opening of a vascular stent, so as to establish a channel for the branch vessel; and the branch opening of the vascular stent is sealed by the sealing stent segment to prevent endoleaks. Moreover, the fixing stent segment may be folded, and is automatically folded after being released from a delivery sheath, so as to prevent the branch stent from moving. In addition, the skirt structure is configured to provide with the developing structure, so that the state of the skirt structure is displayed after being released from the delivery sheath, which makes it convenient for an operator to observe.

According to another embodiment hereof, the fixing stent segment includes a fixing segment stent and a fixing segment covering film disposed on the fixing segment stent; and the developing structure includes a fixing developing portion disposed on the fixing segment stent and/or an end developing portion disposed on the fixing segment covering film.

According to another embodiment hereof, the fixing developing portion includes a plurality of fixing developing points disposed at spaced circumferential intervals on the fixing segment stent.

According to another embodiment hereof, each of the fixing developing point is fixed on the fixing stent segment by welding or suturing, or each of the fixing developing point is developing wire that is spirally wound on the fixing stent segment.

According to another embodiment hereof, the end developing portion includes a plurality of end developing points disposed at spaced circumferential intervals on the fixing segment covering film, and the end developing points are disposed at an end edge of the fixing segment covering film; or the end developing portion includes developing ring disposed at the end edge of the fixing segment covering film.

According to another embodiment hereof, the sealing stent segment has a conical shape, the sealing stent segment includes a sealing segment stent and a sealing segment covering film disposed on the sealing segment stent, the developing structure further includes a plurality of sealing developing points disposed on the sealing segment stent, and the plurality of sealing developing points are uniformly disposed on the sealing segment stent in the circumferential direction.

According to another embodiment hereof, the sealing segment stent is hooked to the fixing segment stent, and the sealing segment covering film is integrated with the fixing segment covering film.

According to another embodiment hereof, the sealing segment stent is hooked to the main body stent, and the sealing segment covering film is fixedly connected to the main body covering film by hot melting.

According to another embodiment hereof, wire diameters of the sealing segment stent and the fixing segment stent are both smaller than that of the main body stent.

According to another embodiment hereof, the main body stent includes a covered stent segment with the main body covering film and a bare stent segment barely disposed at an end of the main body stent. A reinforcing wire is disposed on the bare stent segment, and is wound on the bare stent segment.

### Brief Description of the Drawings

FIG. 1 is a schematic diagram of an overall structure of a branch stent after being implanted into a branch vessel in embodiment I;
FIG. 2 is a schematic diagram of the overall structure of the branch stent in embodiment I;
FIG. 3 is a schematic diagram of the overall structure of the branch stent in another implementation of embodiment I;
FIG. 4 is a schematic structural diagram of a fixing stent segment in an unfolded state in embodiment I;
FIG. 5 is a schematic structural diagram of the fixing stent segment in a partially folded state in embodiment I;
FIG. 6 is a schematic structural diagram of the fixing stent segment in a completely folded state in embodiment I;
FIG. 7 is a schematic structural diagram of the fixing stent segment in an over-folded state in embodiment I;
FIG. 8 is a top view of the fixing stent segment in a completely folded state in embodiment I;
FIG. 9 is a schematic structural diagram of fixing developing point in embodiment I;
FIG. 10 is a schematic structural diagram of a developing wire in embodiment I;
FIG. 11 is a schematic diagram of fixing developing point in another implementation of embodiment I;
FIG. 12 is a schematic diagram of a bare stent segment of the branch stent in embodiment I;
FIG. 13 is a schematic structural diagram of a reinforcing wire in embodiment I;
FIG. 14 is a schematic structural diagram of a fixing stent segment in an unfolded state in embodiment II;
FIG. 15 is a schematic structural diagram of the fixing stent segment in a partially folded state in embodiment II;
FIG. 16 is a schematic structural diagram of the fixing stent segment in a completely folded state in embodiment II;
FIG. 17 is a schematic structural diagram of the fixing stent segment in an over-folded state in embodiment II;
FIG. 18 is a top view of the fixing stent segment in a completely folded state in embodiment II;
FIG. 19 is a schematic cross-section view of placing a vascular stent in an aorta in embodiment III;
FIG. 20 is a schematic structural diagram of a fixing developing portion in embodiment III;
FIG. 21 is a schematic structural diagram of an end developing portion in embodiment III;
FIG. 22 is a schematic structural diagram of a developing ring in embodiment III;
FIG. 23 is a schematic structural diagram of a fixing developing portion in embodiment IV;
FIG. 24 is a schematic structural diagram of an end developing portion in embodiment IV

### Detailed Description

Hereinafter, exemplary embodiments will be described in detail with reference to the accompanying drawings. While exemplary implementations are shown in the drawings, it should be understood that the embodiments and implementations can be embodied in various forms and should not be limited to those set forth herein. Rather, these implementations and embodiments are provided for thorough understanding, and will fully convey the scope of the embodiments to those skilled in the art.

It is to be understood that the terms used herein are for the purpose of describing particular exemplary implementations only and are not intended to be limiting. As used herein, the singular forms "a", "an" and "the" are intended to include the plural forms as well, unless the context clearly indicates otherwise.

Although the terms first, second, third, etc. may be used in the text to describe a number of elements, components, regions, layers, and/or sections, these elements, components, regions, layers, and/or sections should not be limited by these terms. These terms can only be used to distinguish one element, component, region, layer, or segment from another region, layer, or segment. Unless explicitly stated, terms such as "first", "second", and other numerical terms used in the text do not imply sequence or order.

For ease of description, spatial relative relationship terms, such as, "internal", "external", "inner side", "outer side", "under", "below", "on", and "above", may be used herein to describe the relationship of one element or feature relative to another element or feature as illustrated in the figures. These spatially relative terms are intended to encompass different orientations of the device in use or operation in addition to the orientation depicted in the figures. For example, if the device in the figures is turned over, elements described as "below" or "beneath" other elements or features would then be oriented "on" or "above" the other elements or features.

For the ease of description, the following description uses the terms "distal" and "proximal ", where the "distal " refers to positions far away from a heart, and the "proximal " refers to positions close to the heart. The phrase "axial direction" should be understood to indicate a direction in which an interventional device is pushed in and out, and a direction perpendicular to the "axial direction" is defined as the "radial direction".

### Embodiment I

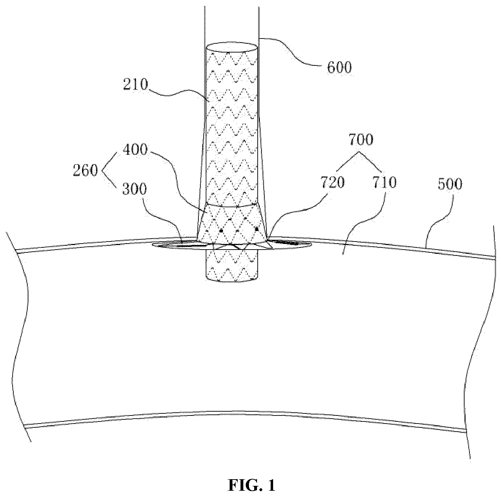

Embodiment I provides a branch stent. As shown in FIGS. 1 to 3, the branch stent 100 includes a stent body 200 and a skirt structure 260 disposed on the stent body 200; the stent body 200 includes a main body stent 210 and a main body covering film 220 disposed on the main body stent 210, where the skirt structure 260 includes a sealing stent segment 400 connected to the stent body and a fixing stent segment 300 connected to the sealing stent segment 400, and the skirt structure 260 is provided with a developing structure 270, and the sealing stent segment 400 has a conical shape.

At present, there are two main application scenarios for the branch stent with the skirt structure. One application scenario is a foldable branch stent for fenestration surgery, which achieves effects of sealing and anchoring by the foldable skirt structure, and the other application scenario is application of the branch stent for chimney surgery. In the embodiment, the branch stent is the foldable branch stent for the fenestration surgery.

An expansion state of a skirt directly determines the endoleaks prevention effect of the branch stent 100. If the skirt structure 260 of the branch stent 100 is not expanded well during implantation, there will be a problem of poor sealing, and even displacement of the branch stent 100. In the embodiment, arranging the developing structure 270 on the skirt structure 260 achieves visualization of the skirt structure 260 during implantation of the branch stent 100, so as to assist in operation of a doctor. The doctor can observe a state of the skirt structure 260 for targeted operation, thereby reducing difficulty and duration of a surgery.

In addition, due to arrangement of the developing structure 270 on the skirt structure 260, the doctor may observe visually whether the skirt structure 260 is placed in expected place, so as to ensure that the skirt structure 260 has good sealing performance and anchoring performance after the branch stent 100 is implanted, and it may ensure smooth operation of the surgery and improve reliability of the surgery.

As shown in FIG. 1, a vascular stent 700 placed in an aorta 500 includes a main cavity 710 and a branch opening 720 formed in a side surface of the main cavity 710. The branch stent 100 in the embodiment can be placed at the branch opening 720 of the vascular stent 700, so as to establish a channel for the branch vessel 600. First, the branch opening 720 of the vascular stent 700 is sealed by the sealing stent segment 400 to prevent endoleaks. Next, the fixing stent segment 300 can be folded, and is automatically folded after being released from the delivery sheath, so as to prevent the branch stent 100 from moving.

In addition, the skirt structure 260 is provided with the developing structure 270. The state of the skirt structure 260 is displayed by the developing structure 270 after being released from the delivery sheath, which makes it convenient for the operator to observe, so as to assist in the operator to implant the branch stent 100.

During the fenestration surgery, first, the vascular stent 700 used for being implanted in the aorta 500 is placed at the diseased aorta 500; then, the vascular stent 700 at a branch vessel 600 is opened to form the branch opening 720; and then, the branch stent 100 is placed at the branch opening 720 to establish a channel communicating with the branch vessel 600.

In the embodiment, the branch stent 100 is provided with the foldable skirt structure 260 which is divided into two segments. The first segment is the sealing stent segment 400 in conical shape; and the sealing stent segment 400 can seal openings in different shapes by its taper shape, so as to effectively prevent endoleaks. The second segment is the foldable fixing stent segment 300; and after the skirt structure 260 is released from the delivery sheath, the fixing stent segment 300 can be automatically folded backwards, so as to prevent the branch stent 100 from moving to an interior of the branch vessel 600. When the branch stent 100 is released, the skirt structure 260 is released first; then, the branch stent 100 is retracted as a whole, to make the skirt structure 260 fitted to an inner wall of the vascular stent 700; and then, the branch stent 100 is completely released.

The fixing stent segment 300 includes a fixing segment stent 310 and a fixing segment covering film 320 disposed on the fixing segment stent 310. The developing structure 270 includes a fixing developing portion 330 disposed on the fixing segment stent 310.

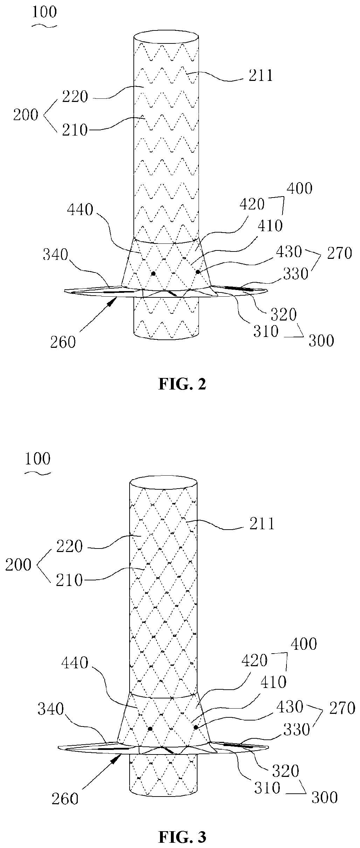

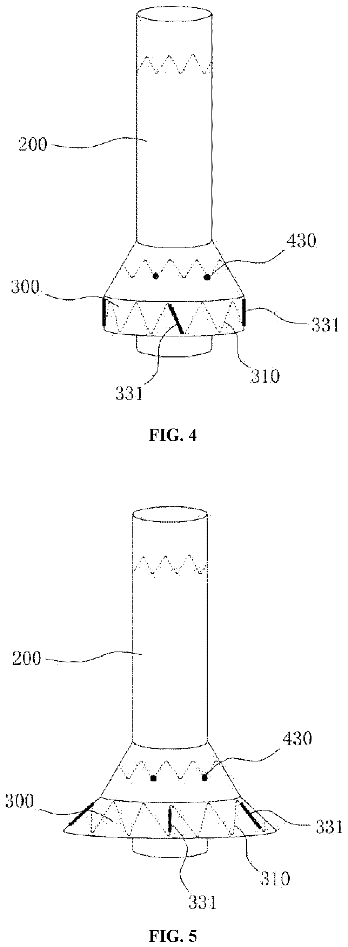

As shown in FIGS. 3 and 4, the fixing developing portion 330 includes a plurality of fixing developing points 331 disposed at spaced circumferential intervals on the fixing segment stent 310. In the embodiment, the plurality of fixing developing points 331 are uniformly disposed on the fixing stent segment 310 in a circumferential direction.

In the embodiment, the fixing stent segment 300 is provided with three to six fixing developing points 331. If there are too few fixing developing points 331, the whole circumferential state of the skirt structure 260 may not be completely displayed. It may be understood that when there are more fixing developing points 331, there may be a more complete circumferential display of the skirt structure 260; however, it may further increase a volume of the branch stent 100, making assembly of the branched support 100 more difficult. Thus, in an embodiment, the number of the fixing developing points 331 is set to four.

The shape of the fixing stent segment 300 is approximately cylindrical, and the fixing developing points 331 are uniformly disposed on a circumference of the skirt.

In an example, as shown in FIG. 4, each fixing developing point 331 is designed into an "I" shape, and four "I"-shaped fixing developing points 331 are symmetrically disposed on the fixing stent segment 300 in the circumferential direction. The released sealing stent segment 400 is configured to has a conical shape, and the released fixing stent segment 300 is approximately in cylindrical shape. One end with a smaller diameter of the sealing stent segment 400 is connected to the stent body 200, and the other end with a larger diameter of the sealing stent segment 400, which can be used for sealing the branch opening 720, is connected to the fixing stent segment 300.

When the fixing stent segment 300 is unfolded, as shown in FIG. 4, at this time, the fixing developing points 331 on the fixing stent segment 300 are observed from the side surface, the fixing developing points 331 in a middle are shown as a complete length, and the fixing developing points 331 at two ends are displayed as a state of being substantially parallel to the stent body 200.

When the fixing stent segment 300 is partially folded, as shown in FIG. 5, at this time, the fixing developing points 331 on the fixing stent segment 300 are observed from the side surface, the fixing developing points 331 in the middle show a shortened length, and the fixing developing points 331 at the two ends are inclined downward.

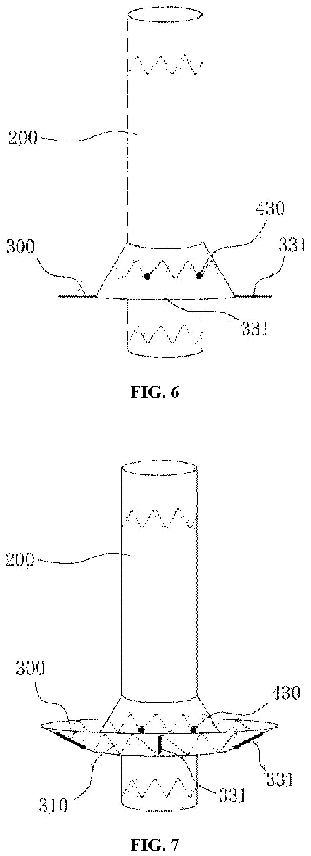

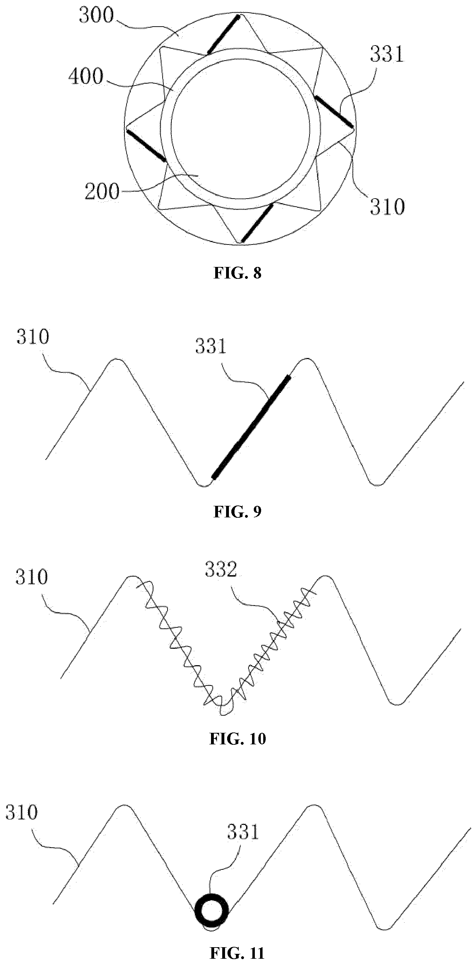

When the fixing stent segment 300 is completely folded, as shown in FIGS.6 and 8, at this time, the fixing developing points 331 on the fixing stent segment 300 are observed from the side surface, the fixing developing points 331 in the middle are shown as a point, and the fixing developing points 331 at the two ends are displayed to be substantially perpendicular to the stent body 200.

When the fixing stent segment 300 is over-folded, as shown in FIG. 7, at this time, the fixing developing points 331 on the fixing stent segment 300 are observed from the side surface, the fixing developing points 331 in the middle show a shortened length, and the fixing developing points 331 at the two ends are inclined upwards.

During the surgery, the change state of the fixing developing points 331 of the fixing stent segment 300 has a clear change order, that is, the unfolded state, the partial folded state, the complete folded state and the over-folded state in sequence. When the skirt structure 260 is retracted and anchored, the fixing developing points 331 of the fixing stent segment 300 are subjected to over-folding, complete folding and partial folding in sequence.

Therefore, during the surgery, the operator can know the folded state of the fixing stent segment 300 at any time by observing a posture change of the fixing developing points 331, so as to avoid mis-operation, or the surgery is terminated in advance before the fixing stent segment 300 has reached a preset shape. If the fixing stent segment 300 is not placed according to the preset posture, it can cause the branch stent 100 to move to an interior of the branch vessel 600 as a whole, and there is a risk of branch endoleaks. In severe cases, there may even be a risk that the branch stent 100 falls off into the branch vessel 600. Through the above solution of the present embodiment, it can be ensured that the fixing stent segment 300 is disposed in a preset posture, so as to avoid the above adverse results.

During observation in the surgery, adjusting the equipment to make any fixing developing points 331 on opposite sides at an approximately maximum interval and observing shapes of the fixing developing points 331 at the two ends later, which the skirt shape at the two ends can be accurately judged. In order to observe the shape of the skirt at other positions, it is only necessary to adjust the equipment to change the fixing developing point 331 from the current position to the two ends. After first adjustment in place, observing the shapes of the skirt at other positions from another angle only needs to rotate the angle of view by 90 degrees, and such observation method is convenient.

Further, as shown in FIGS. 9 to 11, each of the fixing developing points 331 is fixed on the fixing stent segment 300, or each of the fixing developing points 331 is a developing wire 332 spirally wound on the fixing stent segment 300. The fixing developing point 331 can further be in other shapes such as an "O" shape. Additionally, in an embodiment the fixing developing point 331 can be fixed on the fixing stent segment 300 by welding or suturing, or fixed on the fixing stent segment 300 by being wound with the wirelike developing wire 332. The fixing developing point 331 can be made of a tantalum material, a platinum material or other materials having developing performance.

In combination with FIG. 3, the sealing stent segment 400 includes a sealing segment stent 410 and a sealing segment covering film 420 disposed on the sealing segment stent 410. The developing structure 270 further includes a plurality of sealing developing points 430 disposed on the sealing segment stent 410. The number of sealing developing points 430 are uniformly disposed on the sealing segment stent 410 in the circumferential direction.

In the present embodiment, the sealing developing points 430 are disposed on the sealing segment stent 410 for observing a posture of the sealing stent segment 400, which prevent the sealing stent segment 400 from folding driven by a sheath of a delivery device or the vascular stent 700 used for the aorta 500 when the branch stent 100 is released. The fold may lead to a situation where the sealing stent segment 400 cannot seal the branch opening 720, which leads to a failure of the surgery.

The sealing segment stent 410 is partially hooked and connected to the main body stent 210, and is hooked and connected to the fixing segment stent 310; and the sealing segment covering film 420 is integrated with the fixing segment covering film 320, and is fixedly connected to the main body covering film 220 by hot melting.

The main body stent 210 includes a plurality of main body wave coils 211 disposed in sequence; and the plurality of main body wave coils 211 are hooked and connected by adjacent main body wave coils 211. In other embodiments, in combination with FIG. 2, the main body wave coils 211 can further be disposed in a spacing manner, and connected by a stent covering film.

The sealing segment stent 410 includes a plurality of sealing wave coils 440 connected in sequence. The adjacent sealing wave coils 440 are hooked and connected when the number of sealing wave coils 440 is more than one. The sealing stent segment 400 has a conical shape, and the end with a smaller diameter of the sealing stent segment 400 is connected to the main body stent 210. Sealing wave coils 440 is hooked to main body wave coils 211, which makes the sealing stent segment 400 connect to the main body stent 210. In other embodiments, there can further be a preset spacing between the sealing wave coils 440 and the main body wave coils 211, and the sealing segment covering film 420 is connected to the main body covering film 220, so that the sealing stent segment 400 is connected to the main body stent 210.

The fixing segment stent 310 includes a plurality of fixing wave coils 340 connected in sequence.

The adjacent fixing wave coils are hooked and connected when the number of fixing wave coils is more than one. The end with a larger diameter, of the sealing stent segment 400 is connected to the fixing stent segment 300. Sealing wave coils 440 is hooked to fixing wave coils, which makes the sealing stent segment 400 connect to the fixing stent segment 300.The main body wave coils 211, the sealing wave coils 440 and the fixing wave coils are all memory alloy wires. In the present embodiment, a memory alloy nickel-titanium wire is selected. The sealing segment covering film 420 is integrated with the fixing segment covering film 320, and is fixedly connected to the main body covering film 220 by hot melting. In the present embodiment, all covering film structures used are PTFE films.

In the present embodiment, wire diameters of the sealing segment stent 410 and the fixing segment stent 310 are smaller than that of the main body stent 210. By setting the wire diameters of the sealing stent segment 400 and the fixing segment stent 310 to be smaller than that of the main body stent 210, an outside diameter size of the branch stent 100 after compression is reduced, and difficulty of assembly of the branch stent 100 is lowered. The sealing stent segment 400 with smaller wire diameter has less radial supporting force, which means less influence is caused to an inner wall of the branch vessel 600 when the branch stent is implanted in the branch vessel 600. In addition, hardness of the fixing segment stent 310 with the smaller wire diameter is smaller, which is conductive to folding the fixing stent segment 300.

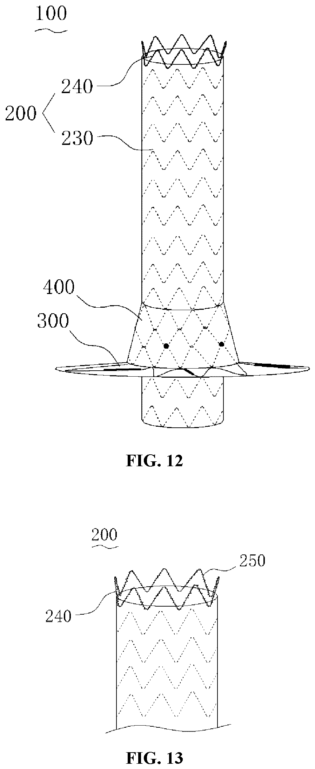

In addition, as shown in FIGS. 12 and 13, the main body stent 210 includes a covered stent segment 230 with the main body covering film 220 and a bare stent segment 240 barely disposed at an end of the main body stent 210. A reinforcing wire 250 is disposed on the bare stent segment 240, and is wound on the bare stent segment 240.

In the present embodiment, winding the reinforcing wire 250 on the bare stent segment 240 can strengthen friction force of the bare stent segment 240, and larger anchoring force can be obtained when the branch stent 100 is loaded into the sheath, which avoid displacement of the branch stent 100 when released from the delivery sheath. The displacement of the branch stent 100 may cause an inaccurate released position.

The reinforcing wire 250 can be the developing wire having the developing performance, so that after the branch stent 100 is released, a position of the end of the branch stent 100 can be determined by observing the reinforcing wire 250 having the developing performance, so as to assist the doctor in implantation of the branch stent 100.

In addition, the doctor can simultaneously observe the relative positions of the reinforcing wire 250 and the sealing developing points 430 disposed on the sealing stent segment 410, so as to determine whether the sealing stent segment 410 is correctly expanded. If the positions of the sealing developing points 430 relative to the reinforcing wire 250 are within a preset range, it indicates that the sealing stent segment 410 is in a correct expansion position.

If the positions of the sealing developing points 430 relative to the reinforcing wire 250 deviate significantly from the preset range, especially if distances of the sealing developing points 430 relative to the reinforcing wire 250 are too short, it indicates that the sealing stent segment 410 is in an over-folded state. The sealing stent segment 410 in the over-folded state cannot effectively seal the opening of the branch stent 100, which will cause a serious medical accident. At this time, it is necessary to adjust the branch stent 100 in time.

Therefore, in the present embodiment, by winding the reinforcing wire 250 on the bare stent segment 240, on one hand, anchoring force between the branch stent 100 and the delivery device can be strengthened, so as to avoid displacement of the branch stent 100 that may cause an inaccurate released position when being released from the delivery sheath. On the other hand, the reinforcing wire 250 can also be used as a position reference for the developing structure 300 to assist the doctor in judging to ensure smooth operation of the surgery.

Thus, the branch stent 100 according to the present embodiment can be placed at the branch opening 720 of the vascular stent 700, so as to establish a channel for the branch vessel 600, and the branch opening 720 of the vascular stent 700 is sealed by the sealing stent segment 400 to prevent endoleaks. The fixing stent segment 300 can be folded, and is automatically folded after being released from the delivery sheath, so as to prevent the branch stent 100 from moving. In addition, the skirt structure 260 is provided with the developing structure 270, which displays the state of the skirt structure 260 after being released from the delivery sheath, making it convenient for the operator to observe.

### Embodiment II

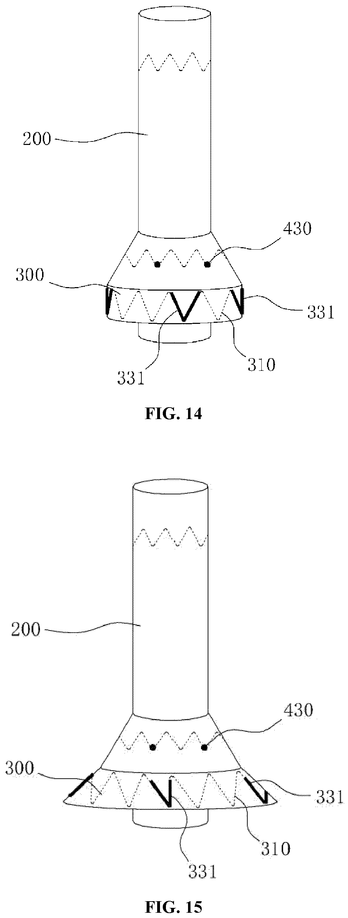

Embodiment II provides a branch stent. As shown in FIG. 14, similarities between embodiment II and embodiment I are no longer repeated. Embodiment II is different from embodiment I, for example, in that fixing developing points 331 can also be designed into a "V" shape, and observation and judgment are the same as those of the fixing developing points 331 in the "I" shape.

For example, if the fixing developing points 331 on a fixing stent segment 300 are in the "V" shape, similarly, after equipment is adjusted first to make any fixing developing points 331 on opposite sides at an approximately maximum spacing, shapes of the fixing developing points 331 at two ends are observed, and then, a shape of the fixing stent segment 300 can be accurately judged.

When the fixing stent segment 300 is unfolded, as shown in FIG. 14, at this time, if the fixing developing points 331 on the fixing stent segment 300 are observed from a side surface, the fixing developing point 331 in the middle shows a complete "V" shape, and the fixing developing points 331 at two ends show a "V" shape with a decreased included angle.

When the fixing stent segment 300 is partially unfolded, as shown in FIG. 15, at this time, if the fixing developing points 331 on the fixing stent segment 300 are observed from the side surface, the fixing developing point 331 in the middle shows a shortened "V" shape, and the fixing developing points 331 at two ends show a "V" shape with a decreased included angle.

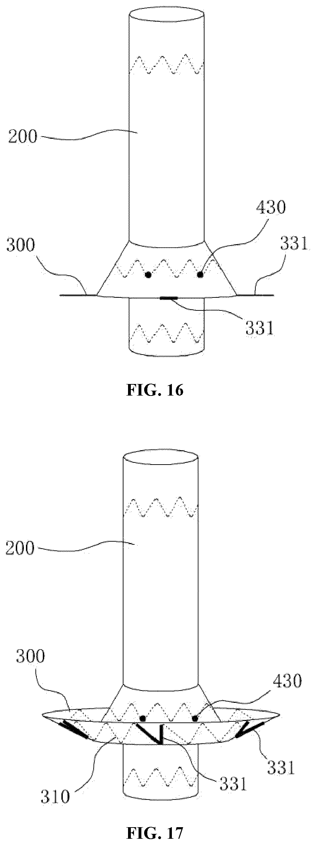

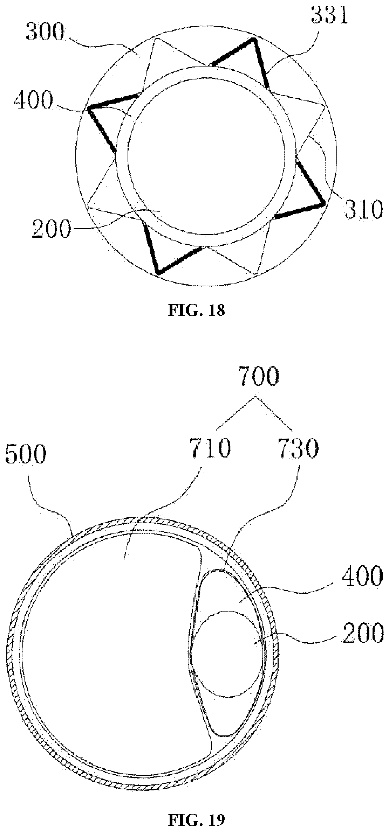

When the fixing stent segment 300 is completely folded, as shown in FIGS. 16 and 18, at this time, the fixing developing points 331 on the fixing stent segment 300 are observed from the side surface, the fixing developing points 331 in the middle are reduced to a point, and the fixing developing points 331 at the two ends are displayed in a strip shape and are substantially perpendicular to a stent body 200.

When the fixing stent segment 300 is over-folded, as shown in FIG. 17, at this time, if the fixing developing points 331 on the fixing stent segment 300 are observed from the side surface, the fixing developing point 331 in the middle shows an inverted "V" shape, and the fixing developing points 331 at two ends show a "V" shape with a decreased included angle.

An operator can judge a current posture of the fixing stent segment 300 by the different states of the fixing developing points 331, and know a folding state of the fixing stent segment 300 at any time, so as to facilitate subsequent operation and ensure a success rate of the surgery.

In other embodiments, the fixing developing points 331 can also be in any other shapes whose changes can be observed, for example, a shape of a number eight, a shape of a number zero and other structure arrangements.

### Embodiment III

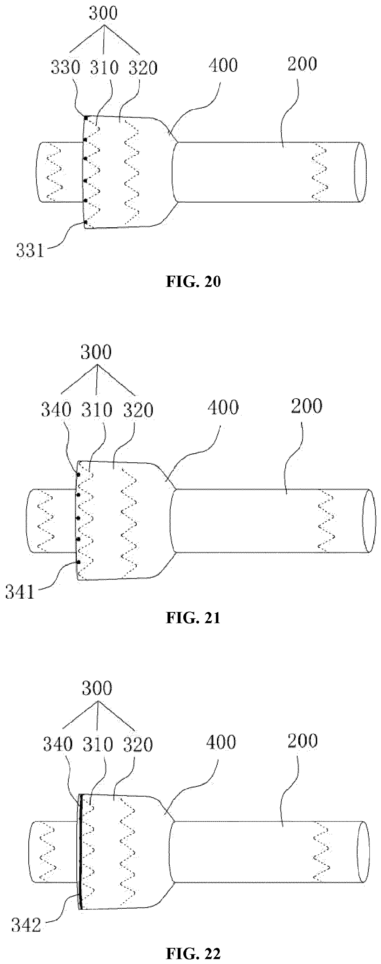

Embodiment III provides a branch stent. As shown in FIGS. 19 and 20, similarities between embodiment III and embodiment I are no longer repeated. Embodiment III is different from embodiment I, for example, in that a developing structure 270 includes a fixing developing portion 330 disposed on a fixing segment stent 310 and/or an end developing portion 340 disposed on a fixing segment covering film 320.

At present, the branch stent 100 is not only applied to a fenestration surgery, but also to a chimney surgery. In the present embodiment, the branch stent 100 is applied to the chimney surgery. In the chimney surgery, a vascular stent 700 for an aorta 500 includes a main cavity 710 and a branch cavity 730 disposed on one side of the main cavity 710. The branch cavity 730 can be round or oval. In a special circumstance, the branch cavity 730 can also be in a diamond or other irregular shapes, where the branch stent 100 is disposed in the branch cavity 730, and a skirt structure 260 on the branch stent 100 mainly plays a blocking role to prevent endoleaks. Therefore, in the chimney surgery, whether the skirt structure 260 is completely expanded and adhered to a wall is a key to determine whether the surgery is successful. Therefore, sealing of the skirt structure 260 is one of important problems that a doctor cares about.

During the surgery, the vascular stent 700 for the aorta 500 is first placed at the diseased aorta 500; then, the branch stent 100 is placed at an opening in a side surface of the vascular stent 700, i.e. the branch cavity 730, and a distal end of the branch stent 100 is connected to a branch vessel 600 to establish a channel, where an edge of the skirt structure 260 of the branch stent 100 needs to be roughly aligned with an edge of a covering film of the vascular stent 700 for the aorta 500.

Therefore, as shown in FIGS. 20 to 21, in the present embodiment, the skirt structure 260 is provided with the developing structure 270. For example, the fixing segment stent 310 is provided with the fixing developing portion 330, or the fixing segment covering film 320 is provided with the end developing portion 340, or the fixing segment stent 310 is provided with the fixing developing portion 330, and the fixing segment covering film 320 is also provided with the end developing portion 340.

In the present embodiment, the fixing developing portion 330 disposed at fixing segment stent 310 is close to the end edge of fixing stent segment 300, to display a position of the end edge of the fixing stent segment 300. The fixing developing portion 330 includes a plurality of fixing developing points 331 disposed at spaced circumferential intervals on the fixing segment stent 310. In the present embodiment, the plurality of fixing developing points 331 are uniformly disposed on the fixing segment stent 310 in the circumferential direction.

The fixing developing point 331 can be fixed on the fixing segment stent 310 by welding or suturing, or fixed on the fixing segment stent 310 by being wound with the developing wire 332.

The end developing portion 340 includes a plurality of end developing points 341 disposed at spaced circumferential intervals on the fixing segment covering film 320; and the end developing points 341 are disposed at the end edge of the fixing segment covering film 320, so as to display the position of the end edge of the fixing stent segment 300, where the end developing points 341 are sutured on the fixing segment covering film 320. In the present embodiment, the plurality of end developing points 341 are uniformly disposed on the edge of the fixing segment covering film 320 in the circumferential direction.

In other embodiments, as shown in FIG. 22, the end developing portion 340 can further include a developing ring 342 disposed on the end edge of the fixing segment covering film 320; and the position of the end edge of the fixing stent segment 300 is displayed by the developing ring 342, where the developing ring 342 is sutured on the fixing segment covering film 320.

Thus, in the present embodiment, the shape of the expanded skirt structure 260 is displayed by the fixing developing portion 330 on the fixing segment stent 310 and/or the end developing portion 340 disposed on the fixing segment covering film 320, so that an operator can visually see whether the skirt structure 260 seals the branch cavity 730 after the branch stent 100 is released, to assist in subsequent operation and ensure the successful operation of the surgery.

### Embodiment IV

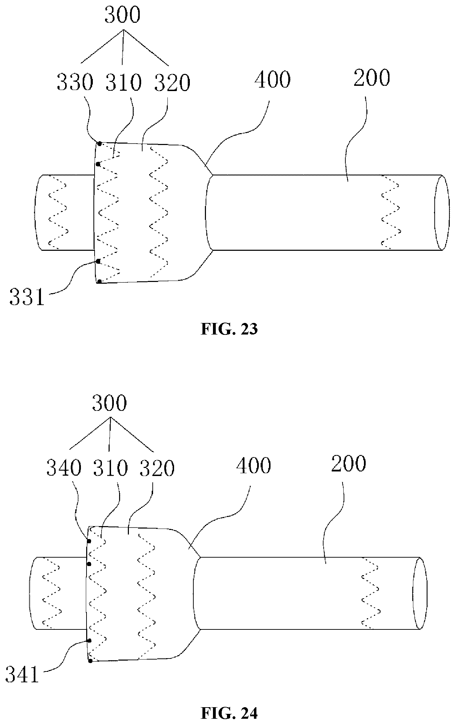

Embodiment IV provides a branch stent 100. As shown in FIGS. 23 and 24, similarities between embodiment IV and embodiment III are no longer repeated. Embodiment IV is different from embodiment III, for example, in that a fixing developing portion 330 includes fixing developing points 331 disposed at two sides of a fixing segment stent 310; and one or more fixing developing points 331 can be disposed at each side. Alternatively, an end developing portion 340 includes end developing points 341 disposed on two sides of a fixing segment covering film 320; and one or more end developing points 341 may be disposed at each side.

The embodiment is suitable for an oval branch cavity 730. As the branch stent 100 will fit to an inner wall of the branch cavity 730 after being placed into the branch cavity 730, and maintain a same shape as the branch cavity 730, and when the branch cavity 730 is oval, a long axis edge of a cross section of the branch cavity 730 has a risk of poor sealing.

In the present embodiment, by directional development on the skirt structure 260, after the branch stent 100 is compressed, the fixing developing portion 330 or the end developing portion 340 is used for displaying a position of the end of long axis of the skirt structure 260 after being compressed, so as to ensure that the skirt structure 260 of the branch stent 100 and the branch cavity 730 of the vascular stent 700 are sealed to avoid endoleaks.

In a specific implantation process of the branch stent 100, it is necessary to assemble the branch stent 100 according to a specific circumferential direction during assembly of the branch stent 100, so that after the branch stent 100 is released, a circumferential angle is controlled by a delivery device, to enable developing structures 270 on two sides of the skirt structure 260 to be located at end of a long axis of an oval.

According to the present embodiment, the fixing developing portion 330 and the end developing portion 340 for displaying the position are disposed on an end edge of the fixing stent segment 300, which helps a doctor to determine a position of the branch stent 100 and ensure accurate positioning. Besides, the doctor can visually observe a deformation shape of the skirt structure 260 on an end surface, so as to judge whether the skirt structure 260 is completely expanded and adhered to a wall to ensure successful operation of the surgery.

The above are only some specific implementations of the present embodiments, but the scope of the present embodiments are not limited to them. Any changes or replacements that can be easily thought of by a person skilled in the art shall be covered within the scope of the present embodiments.

## Claims

1. A branch stent, comprising: a stent body and a skirt structure disposed on the stent body, wherein the stent body comprises a main body stent and a main body covering film disposed on the main body stent; and the skirt structure comprises a sealing stent segment connected to the stent body and a fixing stent segment connected to the sealing stent segment, and the skirt structure is configured to provide with a developing structure.

2. The branch stent according to claim 1, wherein the fixing stent segment comprises a fixing segment stent and a fixing segment covering film disposed on the fixing segment stent; and the developing structure comprises a fixing developing portion disposed on the fixing segment stent and/or an end developing portion disposed on the fixing segment covering film.

3. The branch stent according to claim 2, wherein the fixing developing portion comprises a plurality of fixing developing points disposed at spaced circumferential intervals on the fixing segment stent.

4. The branch stent according to claim 3, wherein each of the fixing developing points is fixed on the fixing stent segment by welding or suturing, or each of the fixing developing points is developing wire that is spirally wound to the fixing stent segment.

5. The branch stent according to claim 2, wherein the end developing portion comprises a plurality of end developing points disposed at spaced circumferential intervals on the fixing segment covering film, and the end developing points are disposed at an end edge of the fixing segment covering film; or the end developing portion comprises developing ring disposed at the end edge of the fixing segment covering film.

6. The branch stent according to claim 2, wherein the sealing stent segment has a conical shape, the sealing stent segment comprises a sealing segment stent and a sealing segment covering film disposed on the sealing segment stent, the developing structure further comprises a plurality of sealing developing points disposed on the sealing segment stent, and the plurality of sealing developing points are uniformly disposed on the sealing segment stent in a circumferential direction.

7. The branch stent according to claim 6, wherein the sealing segment stent is hooked to the fixing segment stent, and the sealing segment covering film is integrated with the fixing segment covering film.

8. The branch stent according to claim 6, wherein the sealing segment stent is hooked to the main body stent, and the sealing segment covering film is fixedly connected to the main body covering film by hot melting.

9. The branch stent according to claim 6, wherein wire diameter of the sealing segment stent and the fixing segment stent are both smaller than that of the main body stent.

10. The branch stent according to claim 1, wherein the main body stent comprises a covered stent segment provided with the main body covering film and a bare stent segment barely disposed at an end of the main body stent, and a reinforcing wire is disposed on the bare stent segment, and is wound on the bare stent segment.
